# EUROPÄISCHE PATENTSCHRIFT

(11) **EP 0 824 025 B1**
(45) Veröffentlichungstag und Bekanntmachung des Hinweises auf die Patenterteilung: **08.03.2006**
(21) Anmeldenummer: 97114104.9
(22) Anmeldetag: 15.08.1997
(51) Int. Cl.: A61N 2/02

(54) **Behandlungsliege für Magnetfeld- oder biomagnetische Signal-Therapie zur Kopf-, Rücken-, Bein- und Körperbehandlung**
Therapeutic table for treating the head, the back, the legs and the body by means of a magnetic field or biomagnetic signals
Table de traitement pour appliquer une thérapie par champ magnétique ou signaux biomagnétiques à la tête, le dos, les jambes et le corps

(30) Priorität: 15.08.1996 DE 29614050 U
(43) Veröffentlichungstag der Anmeldung: 18.02.1998
(73) Patentinhaber: Muntermann, Axel, 35583 Wetzlar (DE)
(72) Erfinder: Muntermann, Axel, 35583 Wetzlar (DE)
(74) Vertreter: Blumbach - Zinngrebe

(56) Entgegenhaltungen:
- EP-A- 0 039 988
- EP-A- 0 407 006
- WO-A-90/07356
- WO-A-91/16941
- WO-A-92/19317

## Beschreibung

Die Erfindung betrifft eine Behandlungsliege, insbesondere zur Behandlung mit magnetischen und/oder elektromagnetischen Feldern, wie sie bei der herkömmlichen Magnetfeldtherapie oder bei der biomagnetischen Signal-Therapie, vorzugsweise zur Kopf-, Rücken-, Bein- und Körperbehandlung, verwendet wird, mit den Merkmalen des Oberbegriffs des Anspruchs 1.

Die biomagnetische Signal-Therapie wird unter anderem beschrieben in den internationalen Veröffentlichungen WO 91/16941 und WO 92/19317. Neben einer Vielfalt von Vorteilen zeichnet sich diese Therapieform dadurch aus, daß bereits mit relativ geringem apparativem Aufwand große Erfolge bei arthrotischen Beschwerden (degenerativen Gelenkbeschwerden), Tendinosen (degenerativen Band- und Sehnenbeschwerden), rheumatischen Erkrankungen im engeren sowie weiteren Sinn und akuten Verletzungen durch Sport- oder Arbeitsunfälle erzielbar sind. Folglich ist eine weite Verbreitung der Therapiegeräte zur flächendeckenden Behandlung derartiger Krankheitsformen von hohem Interesse. Es soll insbesondere dem Allgemeinmediziner die Möglichkeit geschaffen werden, in der eigenen Praxis zu therapieren.

Der Verwendung dieses Verfahrens stehen jedoch, insbesondere im Hinblick auf die Thorax-Behandlung, Kosten entgegen, die vor allem durch die sachgerechte Lagerung des Patienten während der Behandlung und weniger durch den gerätetechnischen Bedarf verursacht sind.

Aus diesem Grund sowie bedingt durch die räumlichen Gegebenheiten des Allgemeinmediziners scheiden grundsätzlich Behandlungsliegen, wie aus dem Bereich der Computertomographie oder Strahlenbehandlung bekannt, für die vorstehenden Therapien aus.

Der WO-92/19317 ist eine Behandlungsliege mit den Merkmalen des Oberbegriffs des Anspruchs 1 zu entnehmen. Diese zeigt eine ortsfest angeordnete Bahre, die von einer in Längsrichtung der Bahre verfahrbaren, ein Magnetfeld erzeugenden Spule umgeben ist. Diese Anordnung mag für kleine Spulendurchmesser, wie diese zur Behandlung von Extremitäten möglich sind, noch zweckmäßig sein. Wenn jedoch die das Magnetfeld erzeugende Spule einen Durchmesser aufweist, der die Behandlung des Thorax eines erwachsenen Menschen zuläßt, wird die Spule sehr schwer und muß entlang des gesamten Verschiebeweges stabil gehalten werden, was sowohl die Konstruktion des mechanischen Unterbaus als auch der elektrischen Verkabelung sehr aufwendig gestaltet. Desweiteren kann durch die zur Einstellung der korrekten Spulenposition nötige Verschiebung unter Umständen im Bereich des Spulenfeldes magnetisierbares Material erfaßt werden, welches zu einer Beeinträchtigung der Homogenität des Feldes und somit der Behandlungsqualität führt.

Die EP 0039988 zeigt ein Vorrichtung zur Krebstherapie, die eine in einer Röhre angerdnete Helixförmige Spule umfasst und eine gepolsterte Liegefläche für einen Patienten aufweist. Die Liegefläche ist auf einem Schlitten relativ zur Spulenanordnung verfahrbar.

Der Erfindung liegt die Aufgabe zugrunde, die Nachteile des Standes der Technik zu vermeiden und insbesondere im Hinblick auf den zunehmenden Kostendruck im medizinischen Bereich die gattungsgemäße Behandlungsliege dahingehend zu verbessern, daß bei hoher Qualität der Behandlung die notwendigen Kosten für die Behandlungsliege gesenkt und gleichzeitig deren Funktionstüchigkeit und -zuverlässigkeit erhöht werden.

Dieses Ergebnis wird auf höchst überraschende Weise bereits durch die Merkmale des Anspruchs 1 erzielt.

Wird die Spule ortsfest angeordnet und die Liegefläche auf einem Schlitten relativ zur Spule verfahrbar ausgebildet, kann der Bereich des Spulenfeldes von störenden, auf die elektromagnetischen und/oder magnetischen Felder negativ wirkenden Materialien befreit und des weiteren eine dauerhafte stationäre Verkabelung mit den nötigen Steuergeräten vorgenommen werden. Der Schlitten weist an dessen Unterseite eine einzige, vorzugsweise mittig angeordnete Längsführungsschiene auf, die sowohl eine seitliche als auch eine vertikale Führung bereitstellt. Durch Verwendung lediglich einer Führungsschiene gegenüber den herkömmlichen Versionen mit zumindest zwei Führungsschienen können zum einen Kosten gespart werden, und zum anderen wird ein Verkanten beim Ein- und Ausbau der Liege vermieden, wodurch der gesamte Vorgang erheblich vereinfacht wird. Wird die Liegefläche und deren zugehöriger Schlitten gelenkig unterteilt, so daß ein über das Gestell der Behandlungsliege hinaus hervorstehender Teil des Schlittens nach unten geschwenkt werden kann, kann sich der Patient vor der Behandlung von der Fußseite her bequem auf die Liegefläche zunächst setzen und dann legen und nach dem Hochklappen des heruntergeschwenkten Teils einfach in die Behandlungsposition verfahren werden. Hierdurch kann der Platzbedarf für die Behandlung, insbesondere für die Längsabmessungen der Liege, erheblich vermindert werden.

Weist der Schlitten eine im wesentlichen ebene Unterfläche auf, die auf einer stationären Wälzkörperanordnung, vorzugsweise auf einer Rollenanordriung, getragen ist, läßt sich dieser leicht in seiner Lage verändern und ist, wenn die Rollen vorteilhafterweise aus einem elastischen Material bestehen, bereits in vertikaler Richtung gefedert. Derartige Rollen können aus Vollgummi, einem geschäumten Material oder mit Luft zu befüllenden Schlauchreifen bestehen.

Weist die Längsführungsschiene seitliche, sich in Längsrichtung erstreckende Nuten auf, in welchen sich Wälzkörper, vorzugsweise Gummirollen, die sowohl eine seitliche als auch eine vertikale Führung bereitstellen, erstrecken, kann hierdurch eine stets spielfreie und leichte Führung der Liege erreicht und ein Hochklappen bei unsymmetrischer Belastung der Liege ausgeschlossen werden.

In vorteilhafter Weise ist die Wälzkörperanordnung mit Abdeckungen versehen, welche die jeweiligen Wälzkörper mit geringem Spiel umgeben, so daß nach Montage und Justierung der Wälzkörper durch die Abdeckungen sowohl ein Verschmutzen verhindert, das Reinigen erheblich erleichtert und aufgrund der langgestreckten Anordnung der Abdeckungen eine zusätzliche Längsführung beim Ein- und Ausbau des Schlittens gewährleistet wird.

Eine besonders stabile Anordnung ergibt sich, falls unterhalb der felderzeugenden Spule eine Stütze angeordnet ist.

Ein variables Programm für Behandlungsliegen verschiedener Größe, wie etwa für verschiedenartige Anwendungsfälle im humanmedizinischen Bereich, im speziellen in Kinderkliniken, bei der ausschließlichen Behandlung von Extremitäten oder im veterinärmedizinischen Bereich zur Kleintierbehandlung wird kostengünstig und flexibel durch einen im wesentlichen modularen Aufbau des Liegengestells mit einem längeren und einem kürzeren Unterteil sowie einer Spulenbaugruppe möglich. Diese Module können vorteilhaft durch seitliche Längsträger mit entsprechend zugeordneten Längen berandet sein.

Weist die Behandlungsliege einen SteuergeräteAblagekasten auf, der vorzugsweise unterhalb des Schlittens im Liegengestell in seiner Position frei wählbar anordenbar ist, kann weiterer Platz für Gerätetische gespart werden. Besonders vorteilhaft ist es, wenn der SteuergeräteAblagekasten an beliebigem Ort im Liegengestell eingehängt werden kann, so daß der Arzt oder das behandelnde Personal die günstigste Arbeitshaltung einnehmen kann.

Mit seitlichen, neben der Liegefläche des Schlittens angeordneten, zusätzlichen Polsterungen wird nicht nur das Äußere der Liege attraktiver gestaltet, sondern auch der seitliche Zugang für den zu lagernden Patienten vereinfacht. Dieser kann sich zunächst auf die seitliche Polsterung setzen und dann nach hinten oder zur Seite auf die Liegefläche legen.

In erfindungsgemäßer Weise wurde große Sorgfalt dafür aufgewendet, daß zumindest für die von dem Spulenfeld erfaßten Bereich, d. h. zumindest das Gehäuse der Spule, und im wesentlichen der Schlitten mit dessen Liegefläche, keine das Magnetfeld beeinflussenden Materialien verwendet wurden. Folglich erwiesen sich Holz, Kunststoff, verleimte Spanplatten, MDF-Platten als hoch geeignet. Für die Längsführungsschiene zeigte Buchenholz bei geringem Verzug und guter mechanischer Bearbeitbarkeit vorteilhafte Eigenschaften.

In vorteilhafter Weiterbildung ist in Längsrichtung der Behandlungsliege vor und/oder hinter der Spulenanordnung, und vorzugsweise auf beiden Seiten der Spule eine Einrichtung zur Erzeugung von Wärmezonen, angeordnet, die bei Bedarf zuschaltbar ist.

Ist die Spulenanordnung in erfindungsgemäßer weise mit einer, vorzugsweise metallischen Isoliereinrichtung gegen die Einrichtung zur Erzeugung von Wärmezonen abgeschirmt, können insbesondere auch Infrarotstrahlen oder Mikrowellen zur Bildung der Wärmezonen eingesetzt werden , ohne daß sich diese und die von der Spule erzeugten Felder gegenseitig negativ beeinflussen.

Um eine zusätzliche Linderung von Beschwerden zu ermöglichen und/oder das Wohlbefinden des Patienten während der Behandlung zu steigern, haben sich zum Kühlen und/oder Erwärmen wenigstens eines Teils der Liegefläche insbesondere eine zwischen der Liegefläche und dem Schlitten angeordnete oder in der Liegefläche integrierte Schicht aus einem thermisch beeinflußbaren Medium oder andere in der Liegefläche eingebaute Wärmezonenquellen, insbesondere Mikrowellen- und/oder Rotlichtstrahler, als vorteilhaft erwiesen.

In alternativer oder weiterer Ausgestaltung ist vorgesehen, daß die Liegefläche voneinander unabhängige Kammern umfaßt, um den Härtegrad einzelner Liegeflächenzonen, vorzugsweise durch Zuführen oder Ablassen von Luft, je nach Bedarf einzustellen und/oder ein, vorzugsweise im wesentlichen formgetreues Anpassen der Liegefläche an den Körper des Patienten zu ermöglichen.

Um für Patienten während der Behandlung eine entspannende, vorzugsweise wellenförmige oder punktbezogene Massagewirkung zu erzielen, umfaßt die Liegefläche in weiterer oder alternativer Ausgestaltung ein mechanisch oder elektrisch ansteuerbares, schwingungs- und/oder vibrationsfähiges System. Dabei kann es sich insbesondere um in der Liegefläche eingebaute Schwingungsmagnete, Vibratoren, Stangen- oder Pumpensysteme handeln.

In weiterer vorteilhafter Ausgestaltung ist an der Behandlungsliege, vorzugsweise am Gehäuse der Spulenanordnung, eine Anzeigeeinrichtung angeordnet, die beispielsweise mit Hilfe von Leuchtdioden das Anzeigen und Erkennen des jeweiligen Behandlungsstatus ermöglicht.

Die Erfindung wird nachfolgend anhand bevorzugter Ausführungsformen detaillierter beschrieben.

Es zeigen:
- Fig. 1: eine Aufsicht auf die erfindungsgemäße Behandlungsliege von oben,
- Fig. 2: eine Schnittdarstellung durch die in Fig. 1 gezeigte Behandlungsliege entlang der Ebene A-A,
- Fig. 3: eine Aufsicht auf die erfindungsgemäße Behandlungsliege von oben bei abgenommenem Schlitten,
- Fig. 4: eine teilweise aufgebrochene Darstellung der erfindungsgemäßen Behandlungsliege von der Seite,
- Fig. 5: eine Explosionsdarstellung des modularen Aufbaus der erfindungsgemäßen Behandlungsliege.

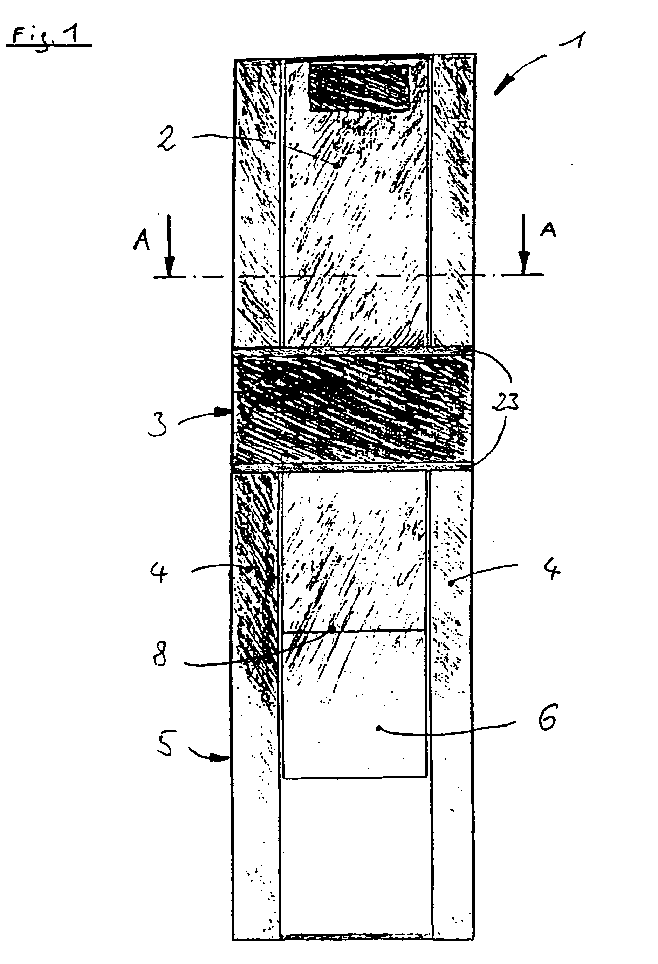

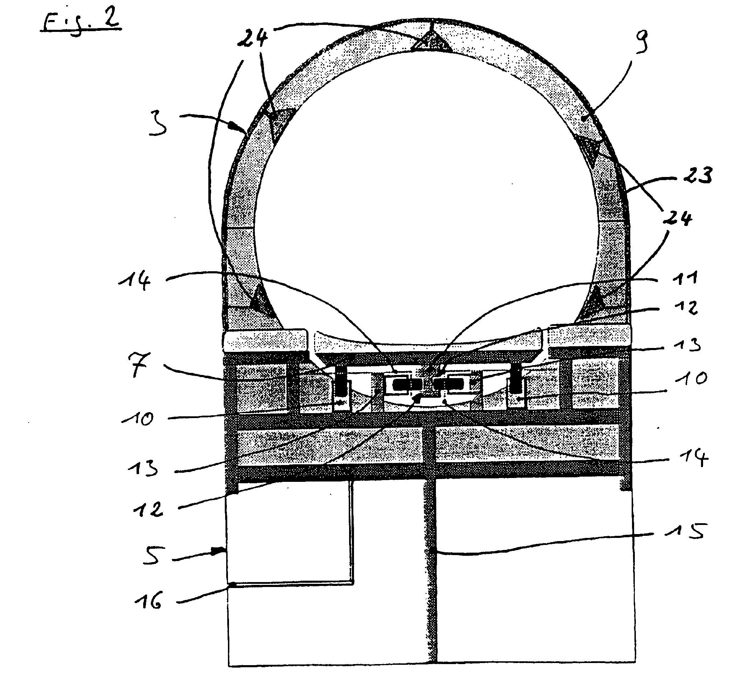

Nachfolgend wird auf Fig. 1 Bezug genommen, in welcher eine erste Ausführungsform der erfindungsgemäßen Behandlungsliege 1 und deren Liegefläche 2 sowie die felderzeugende Spule 3 in einer Aufsicht von oben dargestellt ist. Die seitlichen Polsterungen 4 beranden die Liegefläche und schaffen eine eigenständige Sitzfläche für den zu behandelnden Patienten. Das Liegengestell 5 ist somit nach oben hin fast vollständig gepolstert. Ein Bereich 6 des Fußendes der Liegefläche 2 ist zusammen mit dem zugehörigen Abschnitt des die Liegefläche tragenden Schlittens 7 vermöge eines Gelenkes 8 nach unten verschwenkbar, wenn der Bereich 6 über das Fußende der Behandlungsliege 1 hervorsteht. In dieser Stellung kann ein vom Fußende her auf der Liegefläche 2 sitzender Patient gelegt, sachgerecht positioniert und nach Hochschwenken des Bereichs 6 in die Behandlungslage verschoben werden. Ohne nähere Darstellungen ist es für den Fachmann selbstverständlich, daß der Schlitten 7 durch geeignete Mittel in seiner jeweiligen Stellung arretiert werden kann. Hierzu können Raststellungen definierte Behandlungspositionen markieren oder verriegelnde oder klemmende Eingriffe zwischen Schlitten 7 und Liegengestell 5 vorgesehen sein.

In einer bevorzugten Ausführungsform der Erfindung ist die Spule 3 an deren Stirnseiten mit einer Polsterung 9 versehen, um sowohl Verletzungen bei unsachgemäßer Handhabung zu vermeiden als auch das angenehme Äußere der Liege zu betonen. Für den Patienten angenehm ist weiterhin die doppelkeilförmige Polsterung der Liegenfläche 2.

Die im wesentlichen ebene Unterfläche des Schlittens 7 ist auf einer stationär am Liegengestell 5 befestigten Wälzkörperanordnung 10 getragen. Die Wälzkörper können Kugeln, Walzen oder Rollen sein und sind bei einer bevorzugten Ausführungsform aus elastischem Material, so daß eine zusätzlich Federung sowie eine gewollte definierte Hemmung gegen ein unbeabsichtigtes Verschieben des Schlittens 7 bei darauf angeordnetem Patienten bereitgestellt wird.

In etwa mittig ist in Längsrichtung des Schlittens 7 an dessen Unterseite eine Längsführungsschiene 11 befestigt.

In den Längsseiten der Längsführungsschiene 11 erstrecken sich rechteckförmige Nuten 12, in welchen die Wälzkörper der Wälzkörperanordnungen 13 abrollen. In bevorzugter Weise sind die Wälzkörper der Wälzkörperanordnungen 13 ebenfalls Rollen und stellen innerhalb der Nuten 12 sowohl eine seitliche als auch eine vertikale Führung für den Schlitten 7 bereit. Abdeckungen 14 übergreifen die Wälzkörperanordnungen 10, 13 seitlich und nach oben hin derart, daß nur die Wälzkörper selbst mit geringem seitlichem Spiel hervortreten.

Ohne nähere Darstellung in den Figuren ist es für den Fachmann klar, daß neben definierten Raststellungen des Schlittens 7 sowie Verriegelungsstellungen auch Anschläge vorgesehen sein können, welche entweder jeweilige Endstellungen des Schlittens 7 relativ zum Gestell 5 definieren oder vorbestimmten Behandlungspositionen zugeordnet sind.

Vorzugsweise unterhalb des Spulengehäuses 3 kann im Liegengestell 5 eine weitere Stütze 15 angeordnet sein, die von der Spule ausgeübte vertikale Kräfte aufnimmt. Die Stütze 15 kann sich alternativ auch über die gesamte Länge der Behandlungsliege 1 erstrecken. Seitlich neben der Stütze 15 oder sich bis zur Stütze 15 erstreckend kann ein Steuergerätekasten 16 im Liegengestell 5 in seiner Längsstellung frei wählbar angeordnet werden. Hierzu können dem Fachmann bekannte Einhängeschienen oder auch dauerhafte Befestigungen vorgesehen sein.

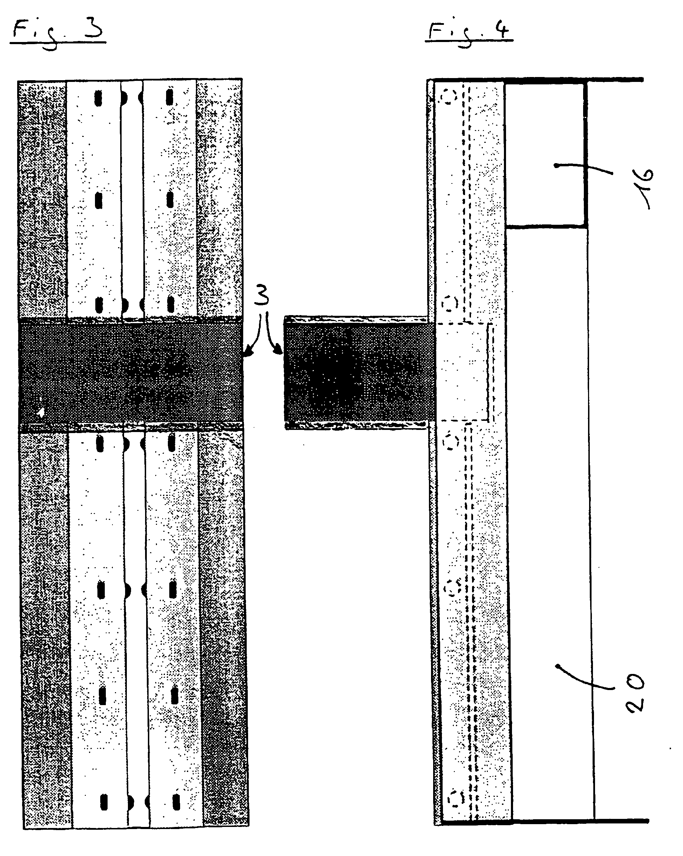

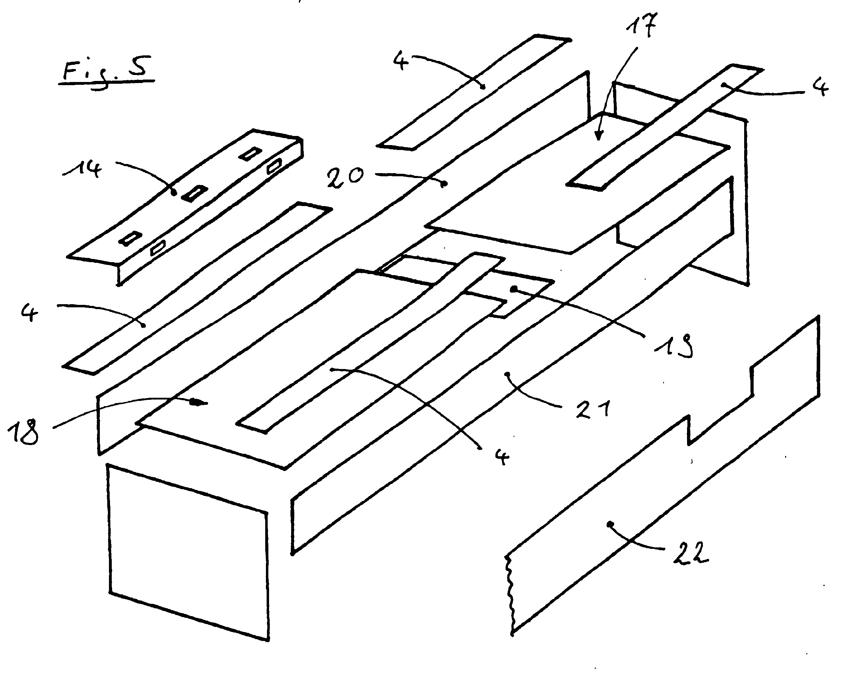

Die erfindungsgemäße Behandlungsliege 1 ist modular aufgebaut und umfaßt, wie besonders gut aus Fig. 5 zu ersehen ist, ein kürzeres und ein längeres Liegenunterteil 17, 18 sowie ein Modul 19 für die Anordnung der Spulenbaugruppe. Seitliche Längsträger 19, 20 beranden die Module 17, 18, 19 und schaffen nach außen ein geschlossenes Bild. Durch geeignete Auswahl der jeweiligen Baugruppen, d. h. der Länge des Liegenunterteils 17, 18, der zugehörigen Polsterungen 4 und der Abdeckungen 14 lassen sich baukastenartig verschiedene Größen der erfindungsgemäßen Behandlungsliege 1 bei geringer Lagerhaltung und standardisiertem kurzem Fertigungsaufwand realisieren.

Mittels eines weiteren Längsträgers 22, der Ausnehmungen zur Aufnahme des Moduls 19 für die Spulenbaugruppe 3 enthält, kann zusätzliche Stabilität geschaffen werden.

In bevorzugter Ausführungsform werden für die Behandlungsliege 1 nur Materialien verwendet, die das Magnetfeld oder ein elektromagnetisches Feld nicht beeinflussen, wie z. B. Holz, Kunststoff, MDF- oder Spanplatten. Als Wicklungskern für die Spule 3 hat sich mit Klebstoff getränkter Karton bewährt, der sowohl geringes Gewicht als auch hohe Stabilität bereitstellt. Am Kopf- oder Fußende des Schlittens 7 können jedoch ohne wesentlichen negativen Einfluß auch metallische Verschraubungen verwendet werden. Bevorzugte Verbindungsverfahren sind jedoch Verleimung, Verzapfung, der Einsatz von Nut- und Federpassungen sowie das Dübeln. Ferner haben sich Schnellmontagesysteme, wie beispielsweise Haken-, Rast- und/oder Stecksysteme, als besonders geeignet erwiesen, mit welchen sich die einzelnen Komponenten der Behandlungsliege auf sehr einfache und leichte Weise zusammenfügen und bei Bedarf wieder voneinander trennen lassen.

Aufgrund des vorstehend beschriebenen modularen Aufbaus der Behandlungsliege liegt es ferner im Rahmen der Erfindung vor und/oder hinter der Spulenanordnung 3 und vorzugsweise an deren beiden Seiten links und rechts der Spule z.B. einen Vorsatz 23 mit, beispielsweise auf Basis von Rot- oder Infrarotlicht oder Mikrowellenenergie, Wärmezonen erzeugenden Strahlern 24 anzuordnen, wie sie insbesondere zur therapeutischen Behandlung von entzündlichen oder rheumatischen Muskelerkrankungen eingesetzt werden. Um zu gewährleisten, daß hierdurch die von der Spule 3 erzeugten Felder nicht beeinflußt werden, kann zwischen den Strahlern 24 und der Spule 3 eine dem Fachmann bekannte, in den Figuren nicht dargestellte, isolierende, vorzugsweise metallische Abschirmung vorgesehen sein. Ist die Spule geeignet abgeschirmt, können Mikrowellen- oder Rotlichtstrahler auch in der Liegefläche integriert sein.

In der Liegefläche 2 oder zwischen der Liegefläche 2 und dem Schlitten 7 kann ferner eine Schicht aus einem thermisch beeinflußbaren Medium integriert bzw. angeordnet sein. Dieses Medium kann zur zusätzlichen Linderung von Beschwerden und/oder zum Wohlbefinden des Patienten auf eine vorwählbare Temperatur heruntergekühlt oder aufgewärmt werden. Geeignete Medien umfassen insbesondere Gele, Öle oder Flüssigkeiten, welche über einen Zeitraum hinweg die vorgewählte Temperatur halten können.

Die Erfindung umfaßt ferner Ausführungsformen, bei denen die Liegefläche mit voneinander unabhängigen, jeweils mit Gas oder Flüssigkeit flutbaren Kammern ausgebildet ist oder wenigstens teilweise, beispielsweise durch in der Liegefläche eingebaute Schwingungsmagnete oder Vibratoren, in einen gewünschten Schwingungs- oder Vibrationszustand versetzt werden kann.

Darüber hinaus kann die Behandlungsliege mit verschieden Anzeigeinstrumenten versehen sein, die vorzugsweise ortsfest am oder im Spulenhäuse 3 oder an einem der seitlichen Längsträger 20, 21 angeordnet sind. Mit Hilfe dieser Anzeigeinstrumente, welche beispielsweise auch Leuchtdioden umfassen können, ist es für die behandelnde Person leicht, Aussagen über den jeweiligen Behandlugsstatus zu treffen, bzw. eine Überprüfung der gewählten Behandlungsmodi vorzunehmen.

## Patentansprüche

1. Behandlungsliege (1) für die Behandlung mit statischen und/oder zeitlich veränderlichen magnetischen oder elektromagnetischen Feldern mit einer felderzeugenden Spulenanordnung und einer Liegefläche für die Lagerung des Körpers eines Patienten, bei welcher sich die Liegefläche (2) durch die felderzeugende Spulenanordnung (3) erstreckt,
die Spulenanordnung (3) ortsfest angeordnet ist und die Liegefläche (2) auf einem Schlitten (7) relativ zur felderzeugenden Spulenanordnung (3) verfahrbar ist, **dadurch gekennzeichnet, daß** der Schlitten (7) der Liegefläche unterseitig eine einzige Längsführungsschiene (11) aufweist, die sowohl eine seitliche als auch eine vertikale Führung bereitstellt.

2. Behandlungsliege nach Anspruch 1,
**dadurch gekennzeichnet, daß** die Liegefläche (2) und der Schlitten (7) gelenkig unterteilt sind, so daß ein über das Liegengestell (5) hinaus hervorstehender Bereich (6) des Schlittens (7) mit der zugehörigen Liegefläche (2) nach unten schwenkbar ist.

3. Behandlungsliege nach Anspruch 1 oder 2,
**dadurch gekennzeichnet, daß** der Schlitten (7) eine im wesentlichen ebene Unterfläche aufweist, die auf einer stationären Wälzkörperanordnung (10), vorzugsweise auf einer elastischen Rollenanordnung getragen ist.

4. Behandlungsliege nach einem der vorstehenden Ansprüche,
**dadurch gekennzeichnet, daß** die Längsführungsschiene (11) seitliche, sich in Längsrichtung erstreckende Nuten (12) aufweist, in welchen sich Wälzkörper, vorzugsweise Gummirollen, erstrecken.

5. Behandlungsliege nach einem der vorstehenden Ansprüche, **dadurch gekennzeichnet, daß** die Wälzkörperanordnungen (10, 13) mit Abdeckungen (14) versehen sind, welche die jeweiligen Wälzkörper mit geringem Spiel umgeben.

6. Behandlungsliege nach einem der vorstehenden Ansprüche, **dadurch gekennzeichnet, daß** unterhalb der felderzeugenden Spulenanordnung (3) eine Stütze (15) angeordnet ist.

7. Behandlungsliege nach einem der vorstehenden Ansprüche umfassend einen im wesentlichen modularer Aufbau des Liegengestells (5), insbesondere mit einem längeren und einem kürzeren Liegenunterteil (17, 18) sowie einer Spulenanordnung (3), welche durch seitliche Längsträger (20., 21) berandet sind.

8. Behandlungsliege nach einem der vorstehenden Ansprüche, **dadurch gekennzeichnet,**
**daß** ein Steuergeräteablagekasten (16), vorzugsweise unterhalb des Schlittens (7), im oder am Liegengestell (5) anordenbar ist.

9. Behandlungsliege nach einem der vorstehenden Ansprüche, **dadurch gekennzeichnet,**
**daß** seitlich neben der Liegefläche (2) des Schlittens (7) eine Polsterung (4) vorgesehen ist.

10. Behandlungsliege nach einem der vorstehenden Ansprüche, **dadurch**
**gekennzeichnet,**
**daß** die Spulenanordnung (3) an deren Stirnflächen eine Polsterung (9) aufweist.

11. Behandlungsliege nach einem der vorstehenden Ansprüche, **dadurch**
**gekennzeichnet,**
**daß** der Schlitten (7) zumindest in dem von der Spulenanordnung (3) umgriffenen Bereich sowie das Gehäuse der Spulenanordnung (3) und der Spulenanordnung (3) benachbarte Bereiche aus Materialien bestehen, die magnetische Felder nicht beeinflussen.

12. Behandlungsliege nach einem der vorstehenden Ansprüche, **gekennzeichnet durch** eine Einrichtung zum Kühlen und/oder Erwärmen der Liegefläche (2).

13. Behandlungsliege nach einem der vorstehenden Ansprüche, **dadurch**
**gekennzeichnet,**
**daß** in Längsrichtung der Behandlungsliege (1) vor und/oder hinter der Spulenanordnung (3) eine Einrichtung zur Erzeugung von Wärmezonen vorgesehen ist.

14. Behandlungsliege nach Anspruch 13, **gekennzeichnet durch** eine Isoliereinrichtung, die die Spulenanordnung (3) gegen die Einrichtung zur Erzeugung von Wärmezonen abschirmt.

15. Behandlungsliege nach einem der vorstehenden Ansprüche, **dadurch**
**gekennzeichnet,**
**daß** an der Behandlungsliege eine Einrichtung zur Anzeige des Behandlungsstatus vorgesehen ist.

## Claims

1. Therapeutic table (1) for treatment with static and / or time-varying magnetic or electromagnetic fields with a field generating coil arrangement and a table area for supporting the body of a patient wherein the table area (2) extends through the field generating coil arrangement (3), the coil arrangement (3) is fixedly arranged and the table area (2) is adapted to be moved on a carriage (7) relative to the field generating coil arrangement (3),
**characterised in that** the carriage (7) of the table area comprises on its lower side a single longitudinal guide track (11) which provides for lateral and vertical guiding.

2. Therapeutic table according to claim 1,
**characterised in that** the table area (2) and the carriage (7) are divided in a jointed way in such a manner that a region (6) of the carriage (7) extending beyond the table frame (5) can be pivoted downwards with the associated table area (2).

3. Therapeutic table according to claim 1 or 2,
**characterised in that** the carriage (7) has an essentially planar lower area which is supported on a stationary rolling element arrangement (10), preferably on an elastic roller arrangement.

4. Therapeutic table according to one of the preceding claims, **characterised in that** the longitudinal guide track (11) comprises lateral grooves (12) extending in the longitudinal direction, in which rolling elements, particularly rubber rollers, extend.

5. Therapeutic table according to one of the preceding claims, **characterised in that** the rolling element arrangements (10, 13) are provided with covering members (14) which surround the respective rolling elements with a small clearance.

6. Therapeutic table according to one of the preceding claims, **characterised in that** a support (15) is arranged below the field generating coil arrangement (3).

7. Therapeutic table according to one of the preceding claims, comprising an essentially modular structure of the table frame (5), particularly with a longer and a shorter table lower part (17, 18) and a coil arrangement (3) which are edged by lateral longitudinal supports (20, 21).

8. Therapeutic table according to one of the preceding claims, **characterised in that**
a control device storage box (16) is to be arranged in or on the table frame (5), preferably below the carriage (7).

9. Therapeutic table according to one of the preceding claims, **characterised in that**
padding (4) is provided to the side beside the table area (2) of the carriage (7).

10. Therapeutic table according to one of the preceding claims, **characterised in that**
the coil arrangement (3) has padding (9) on its end faces.

11. Therapeutic table according to one of the preceding claims, **characterised in that**
the carriage (7) at least in the region contacted by the coil arrangement (3), the housing of the coil arrangement (3) and regions adjacent to the coil arrangement (3) are made of materials which do not influence magnetic fields.

12. Therapeutic table according to one of the preceding claims, **characterised by** a device for cooling and / or heating the therapeutic table (2).

13. Therapeutic table according to one of the preceding claims, **characterised in that**
a device for generating heat zones is provided in the longitudinal direction of the therapeutic table (1) in front of and / or behind the coil arrangement (3).

14. Therapeutic table according to claim 13,
**characterised by** an insulating device which shields the coil arrangement (3) from the device for generating heat zones.

15. Therapeutic table according to one of the preceding claims, **characterised in that**
a device for indicating the status of the treatment is provided on the therapeutic table.

## Revendications

1. Table de traitement (1) pour le traitement à champs magnétiques ou électromagnétiques statiques et/ou variables dans le temps, dotée d'un dispositif de bobines générateur de champ et d'une surface de table pour loger le corps d'un patient, dans laquelle la surface de table (2) s'étend à travers le dispositif de bobines générateur de champ (2), le dispositif de bobines (3) est monté fixe et la surface de table (2) est mobile sur un chariot (7) par rapport au dispositif de bobines générateur de champ (3), **caractérisée en ce que** le chariot (7) de la surface de table comprend sur sa face inférieure un seul rail de guidage longitudinal (11) qui met à disposition un guidage aussi bien latéral que vertical.

2. Table de traitement selon la revendication 1, **caractérisée en ce que** la surface de table (2) et le chariot (7) sont subdivisés de façon articulée, de sorte qu'une zone (6) du chariot (7) dépassant du bâti de table (5) peut pivoter vers le bas avec la surface de table (2) associée.

3. Table de traitement selon la revendication 1 ou 2, **caractérisée en ce que** le chariot (7) comprend une surface inférieure sensiblement régulière qui est supportée sur un dispositif de roulement de palier stationnaire (10), de préférence sur un dispositif de rouleau élastique.

4. Table de traitement selon l'une quelconque des revendications précédentes, **caractérisée en ce que** le rail de guidage longitudinal (11) comprend des rainures (12) latérales, s'étendant dans la direction longitudinale, dans lesquelles s'étendent des roulements de palier, de préférence des rouleaux en caoutchouc.

5. Table de traitement selon l'une quelconque des revendications précédentes, **caractérisée en ce que** les dispositifs de roulement de palier (10, 13) sont pourvus de couvercles (14), lesquels entourent les roulements de palier respectifs avec peu de jeu.

6. Table de traitement selon l'une quelconque des revendications précédentes, **caractérisée en ce qu'**un support (15) est disposé au-dessous du dispositif de bobines générateur de champ (3).

7. Table de traitement selon l'une quelconque des revendications précédentes, comportant une structure sensiblement modulaire du bâti de table (5), dotée notamment de parties inférieures de table longue et courte (17, 18) ainsi que d'un dispositif de bobines (3), lesquels sont bordés par des longerons latéraux (20, 21).

8. Table de traitement selon l'une quelconque des revendications précédentes, **caractérisée en ce qu'**une caisse de réception d'appareil de commande (16) peut être disposée de préférence au-dessous du chariot (7), dans ou sur le bâti de table (5).

9. Table de traitement selon l'une quelconque des revendications précédentes, **caractérisée en ce qu'**un rembourrage (4) est prévu latéralement à côté de la surface de table (2) du chariot (7).

10. Table de traitement selon l'une quelconque des revendications précédentes, **caractérisée en ce que** le dispositif de bobines (3) comprend sur ses surfaces frontales un rembourrage (9).

11. Table de traitement selon l'une quelconque des revendications précédentes, **caractérisée en ce que** le chariot (7) au moins dans la zone entourée par le dispositif de bobines (3) ainsi que le boîtier du dispositif de bobines (3) et les zones voisines du dispositif de bobines (3) sont constitués de matériaux qui n'influencent pas les champs magnétiques.

12. Table de traitement selon l'une quelconque des revendications précédentes, **caractérisée par** un système de refroidissement et/ou de réchauffement de la surface de table (2).

13. Table de traitement selon l'une quelconque des revendications précédentes, **caractérisée en ce qu'**un système destiné à générer des zones chaudes est prévu dans la direction longitudinale de la table de traitement (1) devant et/ou derrière le dispositif de bobines (3).

14. Table de traitement selon la revendication 13, **caractérisée par** un système d'isolement qui protège le dispositif de bobines (3) du système destiné à générer des zones chaudes.

15. Table de traitement selon l'une quelconque des revendications précédentes, **caractérisée en ce qu'**un système destiné à afficher le statut du traitement est prévu sur la table de traitement.
